# EUROPEAN PATENT APPLICATION

(11) **EP 4 664 479 A1**
(43) Date of publication of application: **17.12.2025**
(21) Application number: 25179734.6
(22) Date of filing: 29.05.2025
(51) Int. Cl.: G16H 40/63, G16H 20/40, A61N 5/00

(54) **RADIATION TREATMENT PLAN EVALUATION METHOD AND APPARATUS**

(30) Priority: 13.06.2024 US 202418742106
(71) Applicant: Siemens Healthineers International AG, 6312 Steinhausen (CH)
(72) Inventor: MARKWALDER, Beat, 5619 Buttikon (CH); DONNELLY, Kellee, 00550 Helsinki (FI); McGOOKIN, David, 00180 Helsinki (FI); SABEL, Martin, 6332 Hagendorn (CH); HALKO, Lauri, 00640 Helsinki (FI)
(74) Representative: Mathisen & Macara LLP

(57) **Abstract**

A control circuit (101) accesses (202) a plurality of radiation treatment plans and clinical goals that correspond to radiation treatment planning for a particular patient. The control circuit (101) then utilizes the plurality of radiation treatment plans and the clinical goals within a plan evaluation workspace. The control circuit (101) then presents on a user interface a plan evaluation workspace display (300). This plan evaluation workspace can include, by one approach, both a first area (301) and a second area (302). The first area (301) can discretely present each of a plurality of patient volumes. The second area (302) can graphically present both an extent to which at least one of the clinical goals is fulfilled for each of the plurality of patient volumes by a currently considered radiation treatment plan (113) as well as an available clinical goal-fulfillment solution space that is provided by the plan evaluation workspace for at least some of the plurality of patient volumes.

## Description

### TECHNICAL FIELD

These teachings relate generally to treating a patient's planning target volume with energy pursuant to an energy-based treatment plan and more particularly to facilitating the evaluation of an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Multi-criteria optimization approaches are known and typically require many solutions to be calculated or approximated (for example, pareto front plan collections of tens to hundreds of plans) and often require an abundance of information to be presented well enough for the user to be able to successfully select a most desirable result from such a collection in an understandable way.

### SUMMARY

In accordance with a first aspect of the invention, there is provided a method as defined by claim 1.

In accordance with a second aspect of the invention, there is provided an apparatus as defined by claim 9.

Optional features are defined by the dependent claims.

### BRIEF DESCRIPTION OF DRAWINGS

The above needs are at least partially met through provision of the radiation treatment plan evaluation method and apparatus described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a schematic representation of a user interface as configured in accordance with various embodiments of these teachings;
FIG. 4 comprises a screen shot as configured in accordance with various embodiments of these teachings;
FIG. 5 comprises a screen shot as configured in accordance with various embodiments of these teachings;
FIG. 6 comprises a screen shot as configured in accordance with various embodiments of these teachings; and
FIG. 7 comprises a screen shot as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Multi-criteria optimization/evaluation workspaces often present slider bars that represent a working range of a corresponding plan collection. Each slider bar often corresponds to some alterable optimization/evaluation criteria that the user is able to adjust interactively. In a typical multi-criteria optimization/evaluation workspace, to facilitate developing a radiation treatment plan, the relationship between such adjustable parameters and the corresponding clinical goals is typically indirect. As one example in these regards, a currently selected result is often displayed in numeric form in a variety of different locations on the user's screen. It is up to the user's own exploration, backwards and forwards, to try and develop a reasonable understanding of a current "location" within the plan collection distribution.

Generally speaking, pursuant to these various embodiments, a control circuit that is operably coupled to a user interface is configured to access a plurality of radiation treatment plans and clinical goals that correspond to radiation treatment planning for a particular patient. The control circuit can then utilize the plurality of radiation treatment plans and the clinical goals within a plan evaluation workspace (to aid, for example, in determining and/or outputting an optimized particular radiation treatment plan for that particular patient).

The control circuit can then present on the user interface a plan evaluation workspace display. This plan evaluation workspace can include, by one approach, both a first area and a second area. The first area can discretely present each of a plurality of patient volume quality metrics and corresponding patient volumes. For example, the first area may present each of the plurality of patient volume quality metrics and corresponding patient volumes as an ordered list. The second area can graphically present both an extent to which at least one of the clinical goals is fulfilled for each of the patient volumes (for example, by a given radiation treatment plan) as well as an available clinical goal-fulfillment solution space that is provided by the plan evaluation workspace for at least some of the patient volumes.

In an embodiment, the control circuit may be configured to:
(i) display a current plan indicator indicating an extent to which clinical goals are achieved by a current plan being optimized;
(ii) receive an alteration of the current plan;
(iii) update the plan evaluation workspace display;
(iv) iteratively repeat steps (i)-(iii) until an iteration stop criterion is met; and
(v) when the iteration stop criterion is met, output the altered current plan.

The iteration stop criterion may comprise a user inputting a command to stop the iterative process. Alternatively, the iteration stop criterion may be a particular extent to which the clinical goals are achieved by the current plan.

By one approach, the aforementioned plan evaluation workspace display can also present a graphic current plan indicator.

By one approach, the aforementioned first area can discretely present each of the plurality of patient volume quality metrics on a line-by-line basis. By one approach, in lieu of the foregoing or in combination therewith, the first area can present the plurality of patient volume quality metrics in discrete groups that are segregated by a relative priority. If desired, such segregation is indicated, at least in part, by differing backgrounds (for example, backgrounds that differ with respect to grayscale coloration or that differ with respect to color).

By one approach, the aforementioned second area has a first background treatment to generally demark or demarcate portions that represent unfulfilled clinical goals and a second background treatment that is different from the first background treatment to generally demark or demarcate portions that represent fulfilled clinical goals. If desired, the second area can include a third background treatment that is different from the first background treatment and the second background treatment, and this third background treatment can serve to uniquely indicate the available clinical goal-fulfillment solution space (in, for example, a multi-criteria optimization workspace). These teachings will also accommodate graphically presenting areas within the portion that represents unfulfilled clinical goals that nevertheless constitute an acceptable variation from a corresponding one of the clinical goals. Also if desired, the second area can further include scaled metrics to indicate a degree to which at least one of the clinical goals is fulfilled for each of the patient volumes.

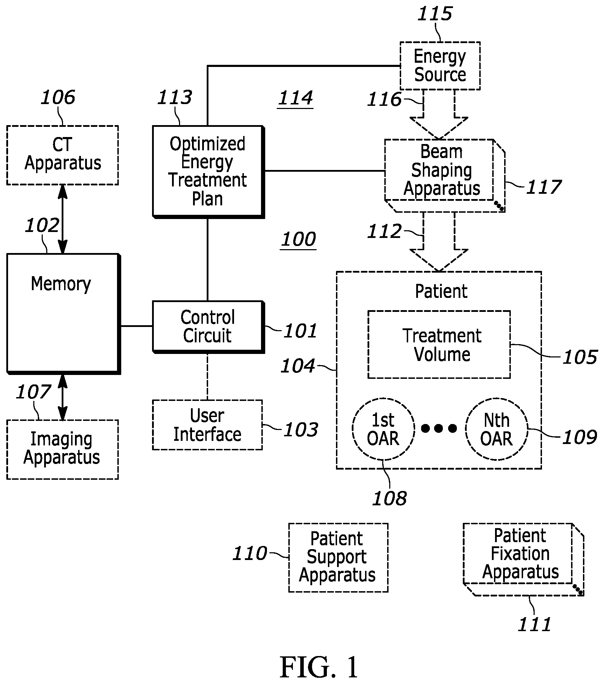

These features and various benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM).)

The control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 can be configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

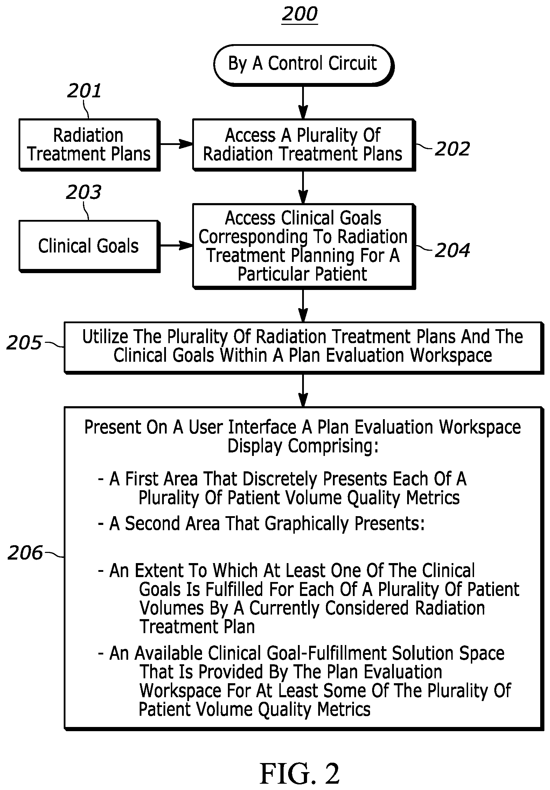

Referring now to FIG. 2, a process 200 that can be carried out, for example, in conjunction with the above-described application setting (and more particularly via the aforementioned control circuit 101) will be described. Generally speaking, this process 200 can serve to facilitate outputting an optimized radiation treatment plan 113 to thereby facilitate treating a particular patient with therapeutic radiation using a particular radiation treatment platform per that optimized radiation treatment plan. More particularly, and for the sake of an illustrative example, this process 200 can serve to facilitate a multi-criteria optimization process. That said, these teachings are also more generally applicable for use in comparing any existing plans (even without a present intent to optimize one or more of those plans) or to assess the quality of a single plan.

At block 202, this process 200 provides for the control circuit 101 accessing a plurality of radiation treatment plans 201 (for example, by accessing the aforementioned memory 102). These radiation treatment plans, in this example, all pertain to treating the same given patient with respect to a same condition (such as a tumor). Generally speaking, multi-criteria optimization employs more than one objective function to be optimized simultaneously with one another. In a typical application setting, at least two of those objective functions conflict with one another. That is, an improvement in one objective function may result in a deterioration of another objective function. As a result, optimizing a radiation treatment plan in this way typically involves making trade-offs between two or more conflicting objectives. (Multi-criteria optimization is known in the art. See, for example, U.S. patent application publication number 2017/0072221 (entitled KNOWLEDGE BASED MULTI-CRITERIA OPTIMIZATION FOR RADIOTHERAPY TREATMENT PLANNING), the contents of which are fully incorporated herein by this reference. Accordingly, further details in these regards are generally not provided here for the sake of brevity.)

At block 204, the control circuit 101 accesses clinical goals corresponding to radiation treatment planning for the aforementioned particular patient (again, and by example, by accessing the aforementioned memory 102). Clinical goals are the treatment goals being prescribed by, for example, the attending oncologist for this particular patient. Examples of clinical goals include, but are not limited to, goals regarding the dose distributions to be achieved with respect to any combination of one or more of: a target volume, one or more organs-at-risk (OAR) in the vicinity of the target volume, and/or other specified or unspecified normal tissues. By their very nature, clinical goals are typically agnostic with respect to what physical radiation treatment platform serves to administer the radiation.

To be clear, it will be understood that clinical goals are not optimization objectives. Optimization objectives provide a measure by which an optimization process can test or assure that, for example, a particular specified dose is being uniformly administered through the patient's target volume while avoiding undue dosing of other patient tissues (or, in other cases, that a series of dose histograms that specify acceptable dosing ranges for a variety of locations both in and external to the target volume are met). Optimization objectives will be understood to be objectives that are very much specifically designed to reflect and accommodate the technical details and specifications of a particular radiation treatment platform, specific details regarding the patient's presentation, and/or other physical details pertaining to a particular application setting.

It will be appreciated that these teachings are highly flexible in practice. As one example in those regards, these teachings can be beneficially applied using other than prescribed clinical goals. For example, these teachings will accommodate accessing essentially any type of evaluation metric that is used as part of a clinician's or planner's review of a given treatment plan. Such evaluation metrics can include any of a variety of quality metrics that may be defined, for example, by a physicist or automatically by the system. One example in these regards would be a metric representing/corresponding to multi-leaf collimator leaf-movement complexity. Accordingly, it will be understood that the expression "clinical goals" as used here in can refer to either prescribed clinical goals or non-prescribed goals.

At block 205, the control circuit 101 utilizes the plurality of radiation treatment plans 201 and the clinical goals 203 within a plan evaluation workspace. As noted above, these teachings will serve well in a variety of plan evaluation workspaces. For the sake of an illustrative example and without intending any limitations in these regards, this description will generally presume that the plan evaluation workspace comprises a multi-criteria optimization workspace.

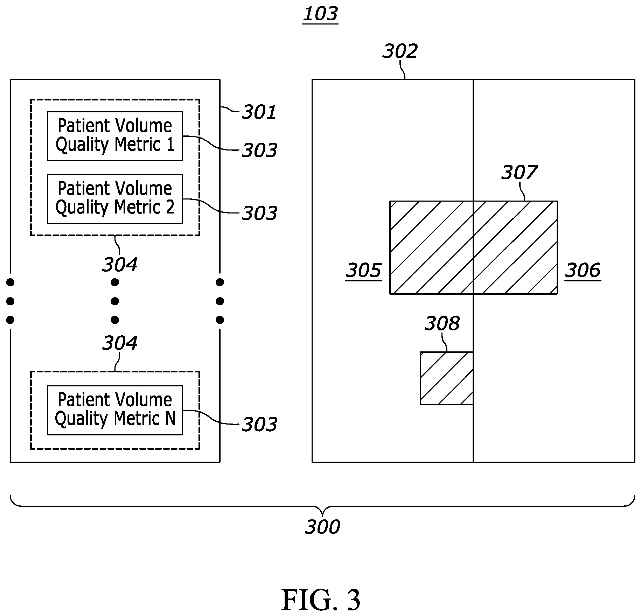

At block 206, and referring as well to FIG. 3, the control circuit 101 presents (in this example, via the above-described user interface 103) a plan evaluation workspace display 300. Generally speaking, this plan evaluation workspace display 300 includes a first area 301 and a second area 302. These teachings will accommodate the inclusion of other areas as well, as desired.

The aforementioned first area 301 serves to discretely, or in the form of a list, present each of a plurality of patient volume quality metrics 303 (illustrated schematically in FIG. 3 by a Patient Volume Quality Metric 1 through a Patient Volume Quality Metric N, where "N" comprises an integer greater than 1). Each of the relevant patient volumes to which these patient volume quality metrics 303 corresponds can comprise target volumes and/or non-targeted volumes (such as organs-at-risk). By one approach, these patient volume quality metrics 303 comprise the aforementioned clinical goals 203 (and/or acceptable variations to those goals) that correspond to each such patient volume.

The foregoing patient volume quality metrics 303 can be presented on a line-by-line basis if desired. For example, this information can be presented on a column-by-column basis, on a row-by-row basis (as illustrated), or by another line-by-line orientation of choice. Other presentation configurations can be accommodated as well as desired.

By one approach, and also as illustrated, the first area 301 can present the plurality of patient volume quality metrics 303 in discrete groups 304 that are segregated by a relative priority. That segregation can be indicated in any of a variety of ways, including by an appropriate use of color, highlighting, icons, and so forth. By one approach, that segregation and grouping is indicated by a use of differing backgrounds (such as, for example, backgrounds that differ by way of a differing grayscale coloration, or that differ in color).

The aforementioned second area 302 graphically presents, for example in the form of a line graph, both (1) an extent to which at least one of the clinical goals 203 is fulfilled for each of the corresponding patient volumes (for example, by a given radiation treatment plan) and (2) an available clinical goal-fulfillment solution space that is provided by the plan evaluation workspace 300 for at least some of the patient volumes.

In support of the foregoing purposes, the second area 302 can have a first background treatment 305 to generally demark or demarcate portions representing unfulfilled clinical goals and a second background treatment 306 that is different from the first background treatment 305 to generally demark or demarcate portions representing fulfilled clinical goals.

These teachings will also support, if desired, providing a third background treatment 307 that is different from the first background treatment 305 and the second background treatment 306 to uniquely indicate the available clinical goal-fulfillment solution space (the latter being represented only schematically in FIG. 3). In a typical application setting, that available clinical goal-fulfillment solution space can vary from one of the patient volume quality metrics 303 to the next.

In lieu of the foregoing, or in combination therewith, these teachings will also support, if desired, the second area further graphically presenting areas 308 within the portion 305 that represent unfulfilled clinical goals, which areas 308 nevertheless constitute an acceptable variation from a corresponding one of the clinical goals 203.

These teachings are highly flexible in practice and will accommodate various modifications and/or supplemental features. As but one example in those regards, the second area 302 can further include scaled metrics to indicate a degree to which at least one of the clinical goals 203 is fulfilled for each of the plurality of patient volumes.

Further details that comport with these teachings will now be presented. It will be understood that the specific details of these examples are intended to serve an illustrative purpose and are not intended to suggest any particular limitations with respect to these teachings.

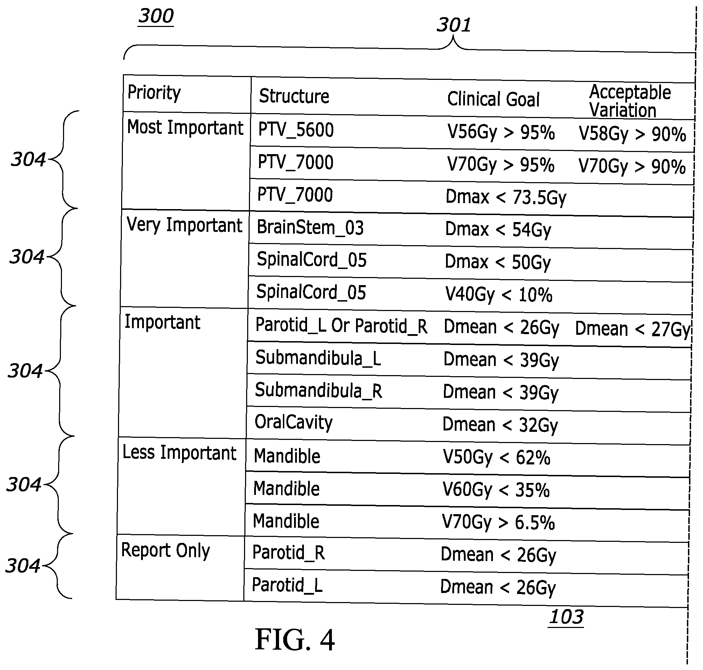

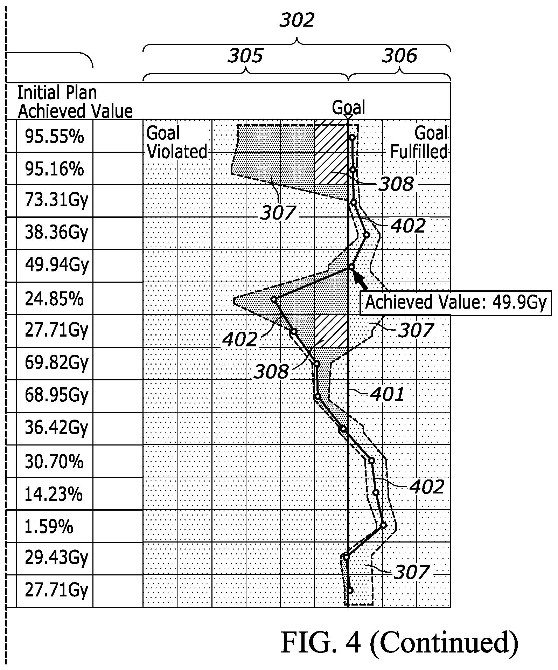

FIG. 4 depicts a user interface 103 that presents a plan evaluation workspace display 300 comprising, in this illustrative example, a multi-criteria optimization workspace for developing a radiation treatment plan. On the left side of the plan evaluation workspace display 300 is the aforementioned first area 301 and on the right side is the second area 302.

Referring to the first area 301, information for each of a plurality of patient volume quality metrics 303 includes identifying information for any combination of one or more of: corresponding patient volumes (such as "PTV_5600" which refers to a particular target volume and "SpinalCord_05" which refers to a non-targeted volume), a corresponding clinical goal for that patient volume (such as a dosing of V56Gy for at least 95% of the PTV_5600 target volume), an acceptable variation from the clinical goal (when such exists), and an achieved dosing value for an initial plan. The plurality of patient volume quality metrics 303 are presented in a line-by-line manner in this example (in this case, in a row-by-row manner).

The first area 301 also groups the foregoing patient volume quality metrics 303 by their corresponding priorities into discrete groups 304. In this illustrative example, the discrete group 304 with the highest priority is at the top, with the prioritization dropping until reaching the lowest priority group 304 at the bottom of the first area 301. To help the user readily perceive these discrete groups 304, a differing background treatment can be presented for each such group 304. In this example, that background treatment comprises grayscale coloration that differs from group to group. These teachings will readily support any of a variety of other approaches in those regards. Examples include, but are not limited to, different colors, differentiating iconography, different fonts or font embellishments, and so forth.

In this example, achieved dosing values are presented in a right-most column for each of the plurality of patient volume quality metrics 303. If desired, values that meet or beat the clinical goal can be visually differentiated from values that fail to meet the clinical goal for a given one of the plurality of patient volume quality metrics 303. For example, a green background can be used behind values that meet/beat the corresponding clinical goal and a red background can be used behind values that fail in those regards.

Referring to the second area 302, in this illustrative example and on the left side of the second area 302 is the aforementioned first background treatment 305 (for example, a red background color treatment) and on the right side of the second area 302 is the aforementioned second background treatment 306 (for example, a green background color treatment). These two areas are separated/delineated by a line 401 that represents the clinical goal for each of the patient volumes. A current plan indicator line 402, in turn, indicates, for each of the patient volumes, an achieved value for each of the plurality of patient volume quality metrics 303 with respect to a corresponding clinical goal. In this example, it can be readily seen that the clinical goal has been met for some, but not all, of the patient volumes, as portions of the current plan indicator line 402 are on the right side of the aforementioned dividing line 401 while other portions are on the left side of that dividing line 401.

In FIG. 4, dashed lines also circumscribe an area having a third background treatment 307 which serves to delineate the available unrestricted clinical goal-fulfillment solution space. This visual delineation of the unrestricted solution space can be a significant aid to the viewer/user when they consider whether, and how, to adjust plan parameters/selections when in search of a better-performing plan. By one approach, this third background treatment 307 can be a color that is visually distinct and contrasting with the background of either or both the first background treatment 305 and/or the second background treatment 306. For example, if the first background treatment 305 is the color red and the second background treatment 306 is the color green, the third background treatment 307 can be a color such as purple, blue, gray, white, and so forth.

FIG. 4 also includes two areas 308 that are shown with cross-hatching. These two areas 308 represent areas that are located within the first background treatment 305 area that represents unfulfilled clinical goals, but which nevertheless constitute an area that represents an acceptable variation from a corresponding one of the clinical goals. (As noted above, the first area 301 of the plan evaluation workspace display 300 can include information for at least some of the plurality of patient volume quality metrics 303 regarding an acceptable variation from a corresponding clinical goal when such exists. These two areas 308 in this example correspond to particular patient volumes having such variation information).

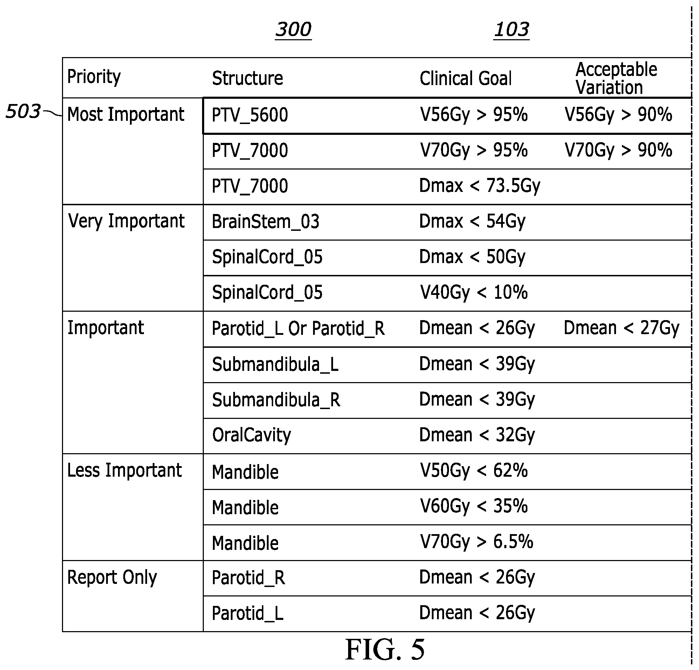

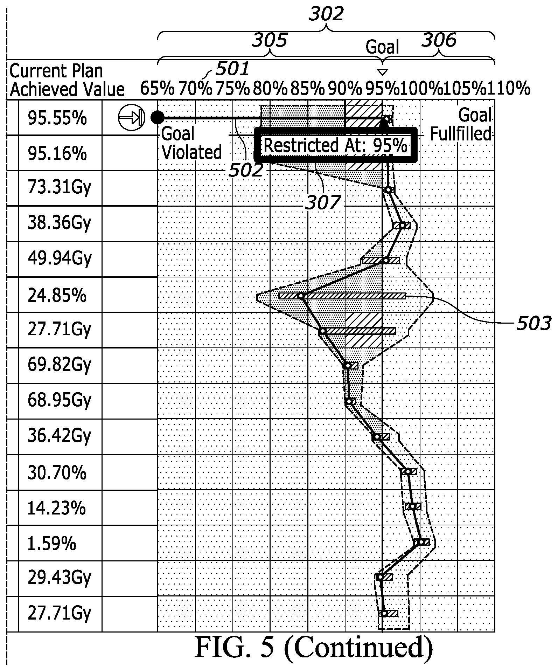

FIG. 5 presents some further attributes that can be included as desired. For the sake of an illustrative example, these further attributes are shown in combination with the example presented in FIG. 4.

In this illustrative example, a series of metrics 501 are presented at the top of the second area 302. In this example, these metrics 501 indicate a particular percentage value (i.e., a percentage of the corresponding clinical goal for each of the plurality of patient volume quality metrics 303) by which the plan achieves, or fails to achieve, a relevant clinical goal. By one approach, and as an example, a corresponding scale 502 can refer to the clinical goal for the PTV-5600 patient volume which appears in the first row 503. That clinical goal is V56Gy >95%, and the aforementioned scale 502 indicates an achieved result of 95.55% with the current plan. By one approach, these teachings can accommodate providing such a display on a metric-by-metric basis for each patient volume quality metric 303 over which the user hovers a cursor.

This illustrative example also includes a series of horizontal bars 503. In particular, in this example there is one such horizontal bar 503 for each of or at least some of the plurality of patient volume quality metrics 303. Each horizontal bar provides a graphic depiction of the available solution space for each of the patient volumes (as versus the unrestricted solution space represented by the third background treatment 307 area).

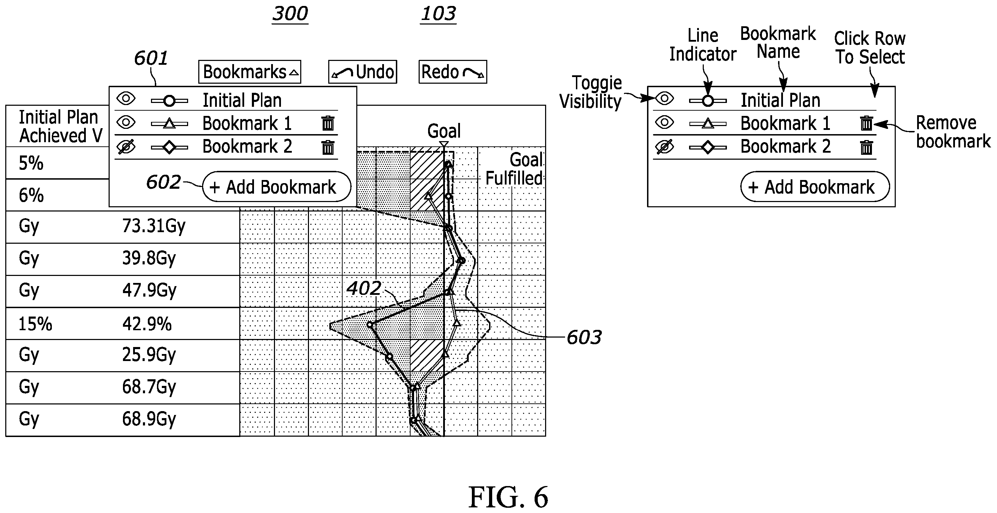

FIG. 6 illustrates that these teachings can be used in conjunction with a bookmarking capability. A user-accessible bookmarking menu 601 can provide an "add bookmark" button 602 to allow a user to bookmark (i.e., save) a particular plan while moving on to make adjustments to test/consider one or more additional plans. In this example, there are three such bookmarks. A first is for the "initial plan" which produced the current plan indicator line 402 described above. A "Bookmark 1" corresponds to a first alteration and has a corresponding dashed line 603 in this example. A "Bookmark 2" corresponds to a second alteration. The resultant plan-results line for the second alteration does not appear because, in this example, the visibility of this bookmark has been toggled "off" by a user.

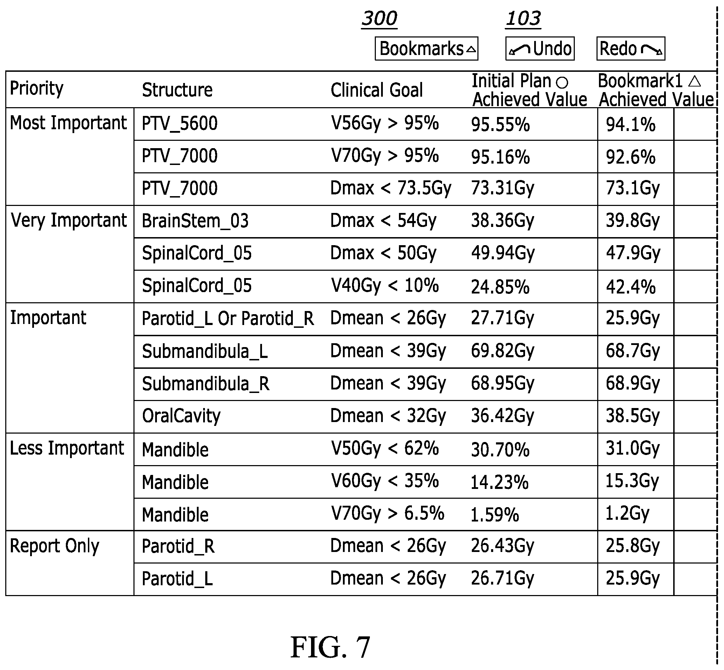

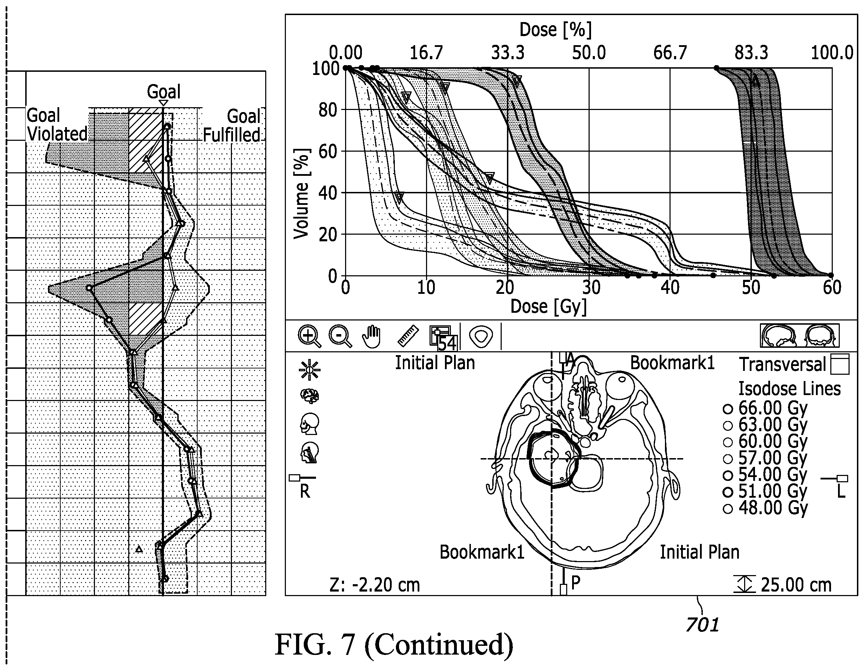

FIG. 7 illustrates that the foregoing plan evaluation workspace display 300 can be simultaneously displayed along with other information such as more traditional content shown as denoted by reference numeral 701.

So configured, these teachings can enable efficiency in the display of information through a condensed graphical representation through simplification of the underlying plurality of plans as a graphical available solution space with respect to clinical goals. These teachings can allow for a clearer understanding regarding which clinical goals can likely reasonably be achieved given the available calculated (or predicted range of) plans. That sense of achievability is typically absent, or at least not easily and readily discernable, in typical prior art solutions.

By one approach a total plan collection can be interpreted as the achievable result for a given patient case. With a typical prior art approach, such a thing is only possible via a user's trial and error approach using sliders. The current teachings help to illuminate what is possible by making clearly visible a range of achievable values for any given clinical goal. The depicted graphic location of current results can readily permit a user to understand the trade-offs between the clinical goals for various ones of the patient volumes by their particular selections.

It will be appreciated that, while multi-criteria optimization in the context of radiation treatment planning typically involves from tens to hundreds of plans, these teachings will support any number of plans, from a single plan to many thousands of plans. In the case of only a single plan, for example, these teachings will provide for readily and easily comparing achieved quality with respect to relevant clinical goals.

Further aspects of the invention are provided by the subject matter of the following clauses:
Clause 1. A method comprising: by a control circuit operably coupled to a user interface: accessing a plurality of radiation treatment plans; accessing clinical goals corresponding to radiation treatment planning for a particular patient; utilizing the plurality of radiation treatment plans and the clinical goals within a plan evaluation workspace; presenting on the user interface a plan evaluation workspace display comprising: - a first area that discretely presents each of a plurality of patient volume quality metrics and corresponding patient volumes; - a second area that graphically presents: - an extent to which at least one of the clinical goals is fulfilled for each of the patient volumes; - an available clinical goal-fulfillment solution space that is provided by the plan evaluation workspace for at least some of the patient volumes.
Clause 2. The method of any combination of the foregoing clauses wherein the first area discretely presents each of the plurality of patient volume quality metrics on a line-by-line basis.
Clause 3. The method of any combination of the foregoing clauses wherein the first area presents the plurality of patient volume quality metrics in discrete groups that are segregated by a relative priority.
Clause 4. The method of any combination of the foregoing clauses wherein the segregation is indicated, at least in part, by differing backgrounds.
Clause 5. The method of any combination of the foregoing clauses wherein the differing backgrounds comprise differing grayscale coloration.
Clause 6. The method of any combination of the foregoing clauses wherein the second area has a first background treatment to generally demark portions representing unfulfilled clinical goals and a second background treatment that is different from the first background treatment to generally demark portions representing fulfilled clinical goals.
Clause 7. The method of any combination of the foregoing clauses wherein the second area has a third background treatment that is different from the first background treatment and the second background treatment to uniquely indicate the available clinical goal-fulfillment solution space.
Clause 8. The method of any combination of the foregoing clauses wherein the second area further graphically presents areas within the portion that represents unfulfilled clinical goals, which areas nevertheless constitute an acceptable variation from a corresponding one of the clinical goals.
Clause 9. The method of any combination of the foregoing clauses wherein the plan evaluation workspace display further comprises a graphic current plan indicator.
Clause 10. The method of any combination of the foregoing clauses wherein the second area further includes scaled metrics to indicate a degree to which at least one of the clinical goals is fulfilled for each of the patient volumes by a currently considered radiation treatment plan.
Clause 11. An apparatus comprising: a memory having a plurality of radiation treatment plans clinical goals corresponding to radiation treatment planning for a particular patient stored therein; a control circuit operably coupled to the memory and to a user interface and configured to: access the plurality of radiation treatment plans; access the clinical goals corresponding to radiation treatment planning for the particular patient; utilize the plurality of radiation treatment plans and the clinical goals within a plan evaluation workspace; present on the user interface a plan evaluation workspace display comprising: - a first area that discretely presents each of a plurality of patient volume quality metrics and corresponding patient volumes; - a second area that graphically presents: - an extent to which at least one of the clinical goals is fulfilled for each of the patient volumes; - an available clinical goal-fulfillment solution space that is provided by the plan evaluation workspace for at least some of the patient volumes.
Clause 12. The apparatus of any combination of the foregoing clauses wherein the first area discretely presents each of the plurality of patient volume quality metrics on a row-by-row basis.
Clause 13. The apparatus of any combination of the foregoing clauses wherein the first area presents the plurality of patient volumes in discrete groups that are segregated by a relative priority.
Clause 14. The apparatus of any combination of the foregoing clauses wherein the segregation is indicated, at least in part, by differing backgrounds.
Clause 15. The apparatus of any combination of the foregoing clauses wherein the differing backgrounds comprise differing grayscale coloration.
Clause 16. The apparatus of any combination of the foregoing clauses wherein the second area has a first background treatment to generally demark portions representing unfulfilled clinical goals and a second background treatment that is different from the first background treatment to generally demark portions representing fulfilled clinical goals.
Clause 17. The apparatus of any combination of the foregoing clauses wherein the second area has a third background treatment that is different from the first background treatment and the second background treatment to uniquely indicate the available clinical goal-fulfillment solution space.
Clause 18. The apparatus of any combination of the foregoing clauses wherein the second area further graphically presents areas within the portion that represents unfulfilled clinical goals, which areas nevertheless constitute an acceptable variation from a corresponding one of the clinical goals.
Clause 19. The apparatus of any combination of the foregoing clauses wherein the plan evaluation workspace display further comprises a graphic current plan indicator.
Clause 20. The apparatus of any combination of the foregoing clauses wherein the second area further includes scaled metrics to indicate a degree to which at least one of the clinical goals is fulfilled for each of the plurality of patient volumes.

Those skilled in the art will recognize that a wide variety of modifications, alterations, and combinations can be made with respect to the above-described embodiments without departing from the scope of the invention. As but one example in these regards, three-dimensional dose distribution predictions from machine learning models (including statical uncertainties or the like) could be overlayed together with a pareto plan collection to assist a user in making clinical trade-offs. Accordingly, it will be understood that such modifications, alterations, and combinations are to be viewed as being within the ambit of the inventive concept.

## Claims

1. A method comprising:
by a control circuit operably coupled to a user interface:
accessing a plurality of radiation treatment plans;
accessing clinical goals corresponding to radiation treatment planning for a particular patient;
utilizing the plurality of radiation treatment plans and the clinical goals within a plan evaluation workspace;
presenting on the user interface a plan evaluation workspace display comprising:
- a first area that discretely presents each of a plurality of patient volume quality metrics and corresponding patient volumes;
- a second area that graphically presents:
- an extent to which at least one of the clinical goals is fulfilled for each of the patient volumes;
- an available clinical goal-fulfillment solution space that is provided by the plan evaluation workspace for at least some of the patient volumes.

2. The method of claim 1 further comprising:
(i) displaying a current plan indicator indicating an extent to which clinical goals are achieved by a current plan being optimized;
(ii) receiving an alteration of the current plan;
(iii) updating the plan evaluation workspace display;
(iv) repeating steps (i)-(iii) until an iteration stop criterion is met; and
(v) when the iteration stop criterion is met, outputting the altered current plan.

3. The method of claim 1 or claim 2 wherein the first area discretely presents each of the plurality of patient volume quality metrics on a line-by-line basis;
and/or:
wherein the first area presents the plurality of patient volume quality metrics in discrete groups that are segregated by a relative priority.

4. The method of claim 3 wherein the segregation is indicated, at least in part, by differing backgrounds;
and/or:
wherein the differing backgrounds comprise differing grayscale coloration.

5. The method of any preceding claim wherein the second area has a first background treatment to generally demark portions representing unfulfilled clinical goals and a second background treatment that is different from the first background treatment to generally demark portions representing fulfilled clinical goals;
and optionally:
wherein the second area has a third background treatment that is different from the first background treatment and the second background treatment to uniquely indicate the available clinical goal-fulfillment solution space.

6. The method of claim 5 wherein the second area further graphically presents areas within the portion that represents unfulfilled clinical goals, which areas nevertheless constitute an acceptable variation from a corresponding one of the clinical goals.

7. The method of any preceding claim wherein the plan evaluation workspace display further comprises a graphic current plan indicator.

8. The method of any preceding claim wherein the second area further includes scaled metrics to indicate a degree to which at least one of the clinical goals is fulfilled for each of the patient volumes.

9. An apparatus comprising:
a memory having a plurality of radiation treatment plans clinical goals corresponding to radiation treatment planning for a particular patient stored therein;
a control circuit operably coupled to the memory and to a user interface and configured to:
access the plurality of radiation treatment plans;
access the clinical goals corresponding to radiation treatment planning for the particular patient;
utilize the plurality of radiation treatment plans and the clinical goals within a plan evaluation workspace;
present on the user interface a plan evaluation workspace display comprising:
- a first area that discretely presents each of a plurality of patient volume quality metrics and corresponding patient volumes;
- a second area that graphically presents:
- an extent to which at least one of the clinical goals is fulfilled for each of the patient volumes;
- an available clinical goal-fulfillment solution space that is provided by the plan evaluation workspace for at least some of the patient volumes.

10. The apparatus of claim 9 wherein the control circuit is further configured to:
(i) display a current plan indicator indicating an extent to which clinical goals are achieved by a current plan being optimized;
(ii) receive an alteration of the current plan;
(iii) update the plan evaluation workspace display;
(iv) repeat steps (i)-(iii) until an iteration stop criterion is met; and
(v) when the iteration stop criterion is met, output the altered current plan.

11. The apparatus of claim 9 or claim 10 wherein the first area discretely presents each of the plurality of patient volume quality metrics on a row-by-row basis;
and/or:
wherein the first area presents the plurality of patient volume quality metrics in discrete groups that are segregated by a relative priority.

12. The apparatus of claim 11 wherein the segregation is indicated, at least in part, by differing backgrounds;
and/or:
wherein the differing backgrounds comprise differing grayscale coloration.

13. The apparatus of any of claim 9 to claim 12 wherein the second area has a first background treatment to generally demark portions representing unfulfilled clinical goals and a second background treatment that is different from the first background treatment to generally demark portions representing fulfilled clinical goals;
and optionally:
wherein the second area has a third background treatment that is different from the first background treatment and the second background treatment to uniquely indicate the available clinical goal-fulfillment solution space.

14. The apparatus of claim 13 wherein the second area further graphically presents areas within the portion that represents unfulfilled clinical goals, which areas nevertheless constitute an acceptable variation from a corresponding one of the clinical goals.

15. The apparatus of any of claim 9 to claim 14 wherein the plan evaluation workspace display further comprises a graphic current plan indicator;
and/or:
wherein the second area further includes scaled metrics to indicate a degree to which at least one of the clinical goals is fulfilled for each of the patient volumes by a currently considered radiation treatment plan.
